(19) Europäisches Patentamt
European Patent Office
Office européen des brevets

(11) **EP 1 230 157 B1**

(12) **EUROPÄISCHE PATENTSCHRIFT**

(45) Veröffentlichungstag und Bekanntmachung des Hinweises auf die Patenterteilung:
**06.08.2003 Patentblatt 2003/32**

(21) Anmeldenummer: **00972734.8**

(22) Anmeldetag: **11.10.2000**

(51) Int Cl.⁷: **C01B 39/40**, C07C 5/27

(86) Internationale Anmeldenummer:
**PCT/EP00/10012**

(87) Internationale Veröffentlichungsnummer:
**WO 01/030697 (03.05.2001 Gazette 2001/18)**

(54) **VERFAHREN ZUR HERSTELLUNG VON SYNTHETISCHEN ZEOLITHEN MIT MFI-STRUKTUR**

METHOD FOR PRODUCTION OF SYNTHETIC ZEOLITES WITH AN MFI STRUCTURE

PROCEDE DE PREPARATION DE ZEOLITHES SYNTHETIQUES A STRUCTURE-MFI (=INDICE D'ECOULEMENT A L'ETAT FONDU)

(84) Benannte Vertragsstaaten:
**AT BE CH CY DE DK ES FI FR GB GR IE IT LI LU MC NL PT SE**

(30) Priorität: **27.10.1999 DE 19951781**

(43) Veröffentlichungstag der Anmeldung:
**14.08.2002 Patentblatt 2002/33**

(73) Patentinhaber: **SÜD-CHEMIE AG**
**80333 München (DE)**

(72) Erfinder:
• **BURGFELS, Götz**
**83043 Bad Aibling (DE)**
• **SCHÖNLINNER, Josef**
**83119 Obing (DE)**
• **SCHMIDT, Friedrich**
**83024 Rosenheim (DE)**

(74) Vertreter: **Reitzner, Bruno, Dr.**
**Splanemann Reitzner**
**Baronetzky Westendorp**
**Patentanwälte**
**Rumfordstrasse 7**
**80469 München (DE)**

(56) Entgegenhaltungen:
EP-A- 0 110 650          EP-A- 0 514 715
EP-A- 0 662 450          WO-A-80/02026
WO-A-93/25476          GB-A- 1 115 489

• DATABASE WPI Section Ch, Week 199727 Derwent Publications Ltd., London, GB; Class E33, AN 1997-296722 XP002158734 & SU 1 610 778 A (BUDOVSKAYA L V), 27. Oktober 1996 (1996-10-27)
• DATABASE CHEMABS [Online] CHEMICAL ABSTRACTS SERVICE, COLUMBUS, OHIO, US; ASAOKA, SACHIO ET AL: "Manufacture of crystalline aluminosilicates" retrieved from STN Database accession no. 106:179031 CA XP002158732 & JP 62 017014 A (CHIYODA CHEMICAL ENGINEERING AND CONSTRUCTION CO., LTD., JAPAN) 26. Januar 1987 (1987-01-26)
• DATABASE CHEMABS [Online] CHEMICAL ABSTRACTS SERVICE, COLUMBUS, OHIO, US; ASAOKA, SACHIO ET AL: "Manufacture of crystalline aluminosilicates" retrieved from STN Database accession no. 106:158848 CA XP002158733 & JP 62 017015 A (CHIYODA CHEMICAL ENGINEERING AND CONSTRUCTION CO., LTD., JAPAN) 26. Januar 1987 (1987-01-26)

**Beschreibung**

Die Erfindung betrifft ein Verfahren zur Herstellung von synthetischen Zeolithen mit MFI-Struktur.

Die DE-A-019 608 660 beschreibt ein Verfahren zur Herstellung von ε-Caprolactam aus Cyclohexanonoxim in der Gasphase unter Verwendung von MFI-Katalysatoren, auf deren Oberfläche sich symmetrisch angeordnete OH-Gruppen befinden. Die Kristallitgröße der MFI-Katalysatoren beträgt < 5 vorzugsweise 0,05 bis 0,5 μm. Die Katalysatoren werden durch hydrothermale Synthese unter Verwendung von Tetrapropylammoniumbromid hergestellt. Über die Verwendung von Impfkristallen finden sich keine Hinweise.

Die EP-B-0 568 566 beschreibt ein Verfahren zur Herstellung von im wesentlichen bindemittelfreien Zeolithen, mit einem $SiO_2/Al_2O_3$-Verhältnis von mehr als 80, wobei bei erhöhter Temperatur ein mit Siliciumdioxid gebundenes Zeolithaggregat in einer wäßrigen ionischen Lösung gealtert wird, welche Hydroxylionen enthält. Man erhält sphärische Kristallite mit einem Durchmesser von < 3μm. Über die Verwendung von Impfkristallen finden sich keine Angaben.

Die EP-B- 0 609 270 beschreibt ein Verfahren zur Herstellung von Zeolithen des MFI-Typs mit gleichmäßiger Kristallgröße, bei dem (i) eine Quelle von teilchenförmigen Siliciumdioxid mit einem mittlerem Durchmesser von 1μm oder weniger; (ii) Impfkristalle vom MFI-Zeolith mit einem mittlerem Durchmesser von 100 nm oder weniger in Form einer kolloidalen Suspension; (iii) ein organische strukturdirigierendes Mittel (Templat) ; und (iv) eine Fluorquelle oder eine Alkalimetallquelle unter Bildung einer wäßrigen synthetischen Mischung umgesetzt werden, wobei die Impfkristalle in einer Menge von 0,05 bis 1.700 Gew.-ppm der Synthesemischung vorhanden sind und die Synthesemischung eine Basizität, ausgedrückt als Molverhältnis von $OH^-/SiO_2$, von weniger als 0,1 aufweist, und die Synthesemischung kristallisieren gelassen wird. Die hergestellten Kristalle haben vorzugsweise einen mittleren Durchmesser oder eine mittlere Länge von 0,3 bis 30 μm.

Die Impfkristalle können z. B. dadurch erhalten werden, daß größere Kristalle in einer Kugelmühle zu kleineren Kristallen zerkleinert werden. Nach den Beispielen werden die Impfkristalle getrennt aus Kieselsäure, TPAOH, NaOH und Wasser hergestellt. Sie enthalten kein Aluminiumoxid, da als Impfkristalle Silicalite verwendet werden. Die Impfkristalle werden von der Mutterlauge abgetrennt, getrennt gewaschen, bis das Waschwasser einen pH-Wert von weniger als 10 hat, und als gebrauchsförmige "kolloidale Impfkristallsuspension" verwendet.

Die EP-B-0 643 671 beschreibt einen kristallinen Tectosilicat-ZSM-5-Zeolith, der im wesentlichen aus nadelförmigen Agglomeraten besteht. Das durchschnittliche Verhältnis von Länge zu Durchmesser dieser Agglomerate beträgt mindestens 2,5, wobei die Durchschnittslänge der Agglomerate gewöhnlich in der Größenordnung von 0,02 bis 10 μm, vorzugsweise von 0,4 bis 5 μm, liegt. Der Zeolith kann dadurch hergestellt werden, daß eine Synthesemischung kristallisiert wird, die (i) eine Siliciumdioxidquelle; (ii) eine Quelle für Alunimium, Gallium, Bor, Eisen, Zink oder Kupfer; oder (iii) eine Quelle für ein einwertiges Kation; und (iv) ein organisches Struktursteuerungsmittel (Templat) enthält. Das Verfahren ist dadurch gekennzeichnet, daß die Synthesemischung ursprünglich 0,05 bis 2 000 ppm Impfkristalle eines MFI-Zeolithen mit einer durchschnittlichen größten Abmessung von nicht mehr als 100 nm enthält.

Die EP-A-0 753 483 beschreibt ein Verfahren zur Herstellung eines MFI-Zeolithen, wobei eine Synthesemischung gebildet wird, die frei von organischen Templaten ist, deren molare Zusammensetzung der des herzustellenden Zeolithen entspricht und die Zeolith-Impfkristalle mit einer größten Abmessung von höchstens 100 nm enthält; das impfkristallhaltige Synthesegemisch wird einer hydrothermalen Behandlung unterzogen. Es wurde festgestellt, daß die Anwesenheit von kolloidalen Impfkristallen in dem Zeolith-Synthesegemisch, ein organisches Templat unnötig macht. Die Kristallitgrößen der MFI-Zeolithe nach den Beispielen liegen zwischen 1,2 und 2,8 μm. Die Kristallite sind sargförmig.

Die WO 97/25272 beschreibt ein Verfahren zur Herstellung eines MFI-Zeolithen mit einer nicht-sphärischen Kristallmorphologie, wobei (a)ein wäßrig-alkalisches Synthesegemisch mit einer Kieselsäurequelle und einem organischen Templat mit (b) einem aktiven Synthesegemisch umgesetzt wird, das entweder (i) eine gealterte wäßrig-alkalische Synthesemischung mit einer Kieselsäurequelle und einem organischen Templat oder (ii) eine aktive Mutterlauge, die sich von einem wäßrigen Synthesegemisch ableitet, das für mindestens eine Kristallisation verwendet wurde und aus der die gebildeten Kristalle entfernt wurden, darstellt, worauf das Gemisch aus (a) und (b) einer hydrothermalen Behandlung unterzogen wird, um die Kristallisation in Gang zu setzen.

Aus der EP-A-0 753 485 ist die Verwendung von Zeolith-Impfkristallen mit einer Teilchengröße von höchstens 100 nm bekannt, um die Zeolith-Kristallisation während der thermischen Behandlung eines Zeolith-Synthesegemischs zu beschleunigen. Die thermische Behandlung dauert nicht länger als 24 Stunden. Das Synthesegemisch enthält höchstens 0,1 Gew.-% Impfkristalle. Es wird ein Zeolith vom ZSM-Typ erzeugt. Die verwendeten Impfkristalle werden speziell hergestellt, d.h. sie sind nicht in der Mutterlauge aus einer vorhergegangenen Synthese enthalten. Da die Impfkristalle die Mutterlauge, in der sie kristallisierten, verließen, weisen sie eine geringere Reaktivität auf.

Aus der EP-A-0 021 675 ist ein kontinuierliches Verfahren zur Herstellung eines kristallinen Zeoliths mit einem "constraint index" zwischen 1 und 11 und einem $SiO_2/Al_2O_3$-Verhältnis von mehr als 12 bekannt, wobei ein Gemisch aus Quellen eines Alkalimetalloxids ($R_2O$), eines Aluminiumoxids, eines Siliciumoxids und Wasser unter Einsatz von geregelten Mengen der Reaktanten umgesetzt wird. Die Umsetzung erfolgt unter Verwendung von Impfkristallen aus dem als Produkt anfallenden Zeolith.

Aus den US-A-5 174 977, 5 174 978, 5 174 981 und 5 209 918 sind Zeolithe vom ZSM-5-Typ und Verfahren zu deren Herstellung bekannt. Als Impfkristalle werden speziell hergestellte Impfkristalle verwendet, die nicht aus der Mutterlauge aus einer vorhergegangenen Synthese stammen.

Gegenüber diesem Stand der Technik lag der Erfindung die Aufgabe zugrunde, Al-reiche synthetische phasenreine Zeolithe mit MFI-Struktur und sphärischen nano-kristallinen Kristalliten herzustellen.

Diese Aufgabe wird erfindungsgemäß gelöst durch ein Verfahren zur Herstellung von synthetischen Zeolithen mit MFI-Struktur, einem Si/Al-Atomverhältnis von etwa 8 bis 45 und sehr kleinen Primärkristalliten, wobei man eine Si-Quelle, eine Al-Quelle und ein organisches Templat unter hydrothermalen Bedingungen miteinander umsetzt, wobei das Si/Al-Atomverhältnis etwa 9 bis 50 und das Verhältnis zwischen längster und kürzester Achse der Primärkristallite etwa 1,0 bis 1,5 : 1 beträgt; das Verfahren ist dadurch gekennzeichnet, daß man die Umsetzung in Gegenwart von Impfkristallen mit einer mittleren Teilchengröße von etwa 10 bis 100 nm, vorzugsweise von 20 bis 50 nm aus einem früheren Ansatz ohne Abtrennung von der Mutterlauge durchführt.

Das erfindungsgemäße Verfahren hat gegenüber dem Stand der Technik den Vorteil, daß die eingesetzten Impfkristalle nicht zusätzlichen Behandlungsschritten unterzogen werden müssen, d.h. die Impfkristalle verlassen in keinem Stadium des Verfahrens den günstigen pH-Bereich von > 11. Durch diese Maßnahme wird die besonders hohe Aktivität der Impfkristalle sichergestellt.

Um die Teilchengröße der Impfkristalle zu bestimmen, wurden die Teilchen mittels einer Zentrifuge von einer Mutterlauge abgetrennt und mit Aceton mehrmals gewaschen. Das erhaltene Produkt wurde getrocknet und anschließend mit dem Rasterelektronenmikroskop untersucht. Dabei konnte eine Teilchengröße von 20 bis 50 nm festgestellt werden. Die Teilchen waren im wesentlichen sphärisch.

Vorzugsweise verwendet man als Si-Quelle Fällungskieselsäure, als Al-Quelle Natriumaluminat und als organisches Templat Tetrapropylammoniumbromid.

Die hydrothermale Umsetzung wird vorzugsweise bei einer Temperatur von etwa 100 bis 180°C, einem pH-Wert von etwa 11 bis 13 und bei einem Gewichtsverhältnis zwischen Si- und Al-Quelle von etwa 2,95 bis 26,5 durchgeführt.

In einer weiteren Behandlungsstufe wird das Reaktionsprodukt vorzugsweise getrocknet und calciniert.

Gegenstand der Erfindung sind ferner synthetische Zeolithe mit MFI-Struktur, die nach dem vorstehend beschriebenen Verfahren erhältlich und durch die Kombination folgender Merkmale gekennzeichnet sind:

(a) Si/Al- Atomverhältnis 8 bis 45 : 1
(b) Größe der Primärkristallite etwa 0,01 bis 0,05 μm;
(c) Verhältnis zwischen längster und kürzester Achse der Primärkristallite: etwa 1,0 bis 1,5 : 1;
(d) Verhältnis zwischen der Intensität der Röntgenbeugungslinie mit der höchsten Intensität ($D_{max} = 3{,}865 \pm 0{,}015$) und der Intensität der Röntgenbeugungslinien von ZSM 11-Zeolith ($D_{max} = 11{,}15$ Å); Mordenit ($D_{max} = 9{,}06$ Å); $\alpha$-Cristobalit ($D_{max} = 3{,}345$ Å) und Analcim ($D_{max} = 4{,}83$ Å) > 500 : 1.

Eine Weiterbildung des erfindungsgemäßen Verfahrens ist dadurch gekennzeichnet, daß man das Reaktionsprodukt durch Ionenaustausch in die H-Form überführt, unter Zusatz von Bindemitteln Formkörper erzeugt und vor oder nach der Erzeugung der Formkörper gegebenenfalls katalytisch wirksame Unedelmetall- und/oder Edelmetallkomponenten zusetzt.

Die erfindungsgemäßen synthetischen Zeolite sind als Katalysatoren verwendbar, wobei insbesondere die Zeolithe in der H-Form (mit oder ohne Belegung mit Unedelmetallen und/oder Edelmetallen) als Katalysatoren für sauer katalysiete Reaktionen, Oxidationen, Reduktionen und Adsorptionen eingesetzt werden können.

Diese Reaktionen umfassen u.a. das katalytische Cracken (FCC-Additiv) und Hydrocracken (auch "Dewaxing" durch schonendes Hydrocracken);
Alkylierungen z. B. von Aromaten mit Olefinen, Alkoholen oder mit halogenhaltigen Paraffinen;
Alkylierung von Aromaten;
Alkylierung von Isoparaffinen mit Olefinen;
Transalkylierung (von Aromaten) ; Disproportionierung (z.B. Toluol-Disproportionierung, usw.); Isomerisierung und Hydroisomerisierung (z.B. von Paraffinen, Olefinen, Aromaten, Xylol-Isomerisierung, Dewaxing, usw.); Dimerisierung und Oligomerisierungen;
Polymerisationen;
Veretherungen und Veresterungen;
Hydratisierung und Dehydratisierung;
Adsorption;
Kondensation;
Oxidation;

Acetalisierung;

Dealkylierung und Cyclisierung.

Alkylierung und Hydrodealkylierung (Ethylbenzol zu Benzol); Abgasreinigung;

Säurekatalysierte Reaktionen sind beispielsweise auch in der DE-A-4 405 876 angegeben, wobei ein Katalysator auf der Basis eines teilchenförmigen säureaktivierten Schichtsilicats verwendet wird, dessen Teilchen durch ein Bindemittel miteinander verbunden sind.

Bei ihrer Verwendung als Katalysatoren liegen die erfindungsgemäßen Produkte im allgemeinen in stückiger Form, z.B. als Extrudate, Granulate, Pellets, Kugeln, Wabenkörper und andere Formkörper vor.

Um eine gewisse Festigkeit zu erzielen, setzt man den Massen zur Herstellung der Formkörper im allgemeinen ein anorganisches, metallorganisches oder organisches Bindemittel zu. Als anorganische Bindemittel verwendet man vorzugsweise Kieselsäure oder Aluminiumhydroxid-Sole. Ferner sind Aluminate, Titanate oder Phosphate geeignet. Besonders geeignet sind Erdalkaliverbindungen, die bei der Reaktion mit Säure schwerlösliche Salze bilden, wobei Strontium- und Bariumverbindungen gegenüber den entsprechenden Calciumverbindungen bessere Bindemittel ergeben.

Weiterhin eignen sich als anorganische Bindemittel Verbindungen der Metalle der Gruppen IIIA (vorzugwsweise $Y_2O_3$), IIIB (vorzugsweise $B_2O_3$, $Al_2O_3$ oder $Al(H_2PO_4)_3$), IVA (vorzugsweise $TiO_2$ und $ZrO_2$), IVB (vorzugsweise Oxide, Carbide oder Nitride des Siliciums, der Lanthanoiden, vorzugsweise $LaO_2$ oder $CeO_2$) der Aktinoiden (vorzugsweise $ThO_2$).

Als anorganische Bindemittel können auch hydraulische Bindemittel, vorzugsweise Zement oder Gips, bzw. die natürlichen Silicatbindemittel, wie Mullit oder Talk, verwendet werden.

Das metallorganische Bindemittel kann eine Verbindung der Formel $Me(OR)_n$ oder der Formel $Me(O-CO-R)_n$ sein, worin Me ein Metall mit einer Wertigkeit n und R einen organischen Rest, z.B. einen Alkyl-, Aryl-, Aralkyl-, Alkaryl- oder hetercyclischen Rest darstellen.

Das organische Bindemittel kann eine natürliches , halbsynthetisches oder synthetisches Polymer bzw. eine Vorstufe hiervon darstellen, das bei den Verarbeitungs- und/oder Anwendungsbedingungen seine Bindeeigenschaften nicht verliert. Als Beispiele seien Alken-(Co)Polymerisate, Polykondensate, Polyadditionsverbindungen, Siliconkautschuk, Siliconharze, Kautschuk, Knochenleim, Casein, Galalith, Alginate, Stärke, Cellulose, Guar, Carboxymethylcellulose, Polyvinylalkohol, Polyacryl- oder Polymethacrylverbindungen sowie Additions- oder Kondensationsharze genannt. Insbesondere können Furanharze oder Phenolharze verwendet werden.

Beispiele für Bindemittel sind in der DE-A-4 405 876 angegeben.

Die anorganischen, metallorganischen und/oder organischen Bindemittel können erfindungsgemäß in geeigneter Weise miteinander kombiniert werden. Hierbei kann es von Vorteil sein, zumindest einen Teil der Bindemittel in ihrer kolloidalen Form und/oder in löslicher Form einzusetzen. Einige Bindemittel können selbst katalytisch aktiv sein.

Das relative Gewichtsverhältnis zwischen dem ausgewählten Zeolithen mit MFI-Struktur einerseits und dem Bindemittel andererseits variiert von etwa 20 bis 80 Gew.-%. Der Zeolith mit MFI-Struktur liegt vorzugsweise in einer Menge von mehr als etwa 50 Gew.-%, insbesondere von mehr als 60 Gew.-%, vor.

Die Erfindung ist durch die nachstehenden Beispiele erläutert.

Beispiel 1

Herstellung von Zeolithen in der Na-Form

1.662 g demineralisiertes Wasser, 5,24 g Natriumhydroxid und 709,5 g Natriumaluminat wurden unter kräftigem Rühren auf 80°C erhitzt, bis eine klare Lösung erhalten wurde, die dann auf unter 60°C abgekühlt wurde.

In einem getrennten Gefäß wurden 20 Liter einer Mutterlauge aus einem früheren Ansatz, die (analytisch) 3,43 Gew.-% $SiO_2$, 2,16 Gew.-% $Na_2O$ und 5,5 Gew.-% Tetrapropylammoniumbromid (TPABr) enthielt, vorgelegt. Die Mutterlauge enthielt ferner etwa 0,1 Gew.-% Impfkristalle mit einer mittleren Teilchengröße von 20 bis 50 nm. Die Impfkristalle hatten folgende (analytische) Zusammensetzung $SiO_2$: 91,77 Gew.-%, $Al_2O_3$ 3,49 Gew.-%; Atomverhältnis Si/Al=26,4

Die Mutterlauge wurde mit etwa 6 Liter demineralisiertem Wasser verdünnt, worauf 1 980g TPABr und 6 260 g Fällungskieselsäure (Silica VN3LCC-Degussa) zugesetzt wurden. Das Gemisch wurde 30 bis 60 min. gerührt, worauf 2 380 g der ersten Lösung zugesetzt wurden.

Das Gemisch wurde etwa 30 bis 60 min. homogenisiert. Der pH-Wert des Gels betrug 12,7.

Das Synthesegemisch wurde in einem 40 Liter-Autoklaven, der mit einem Rührwerk versehen war, eingefüllt und allmählich auf etwa 130°C erhitzt, wobei mit einer Rührerdrehzahl von etwa 73 UpM gerührt wurde. Der Druck stieg während der Reaktion aufgrund der Zersetzung des TPABr allmählich von 2,5 auf 3,5 bar an.

Nach 20 Stunden wurde eine erste Probemenge von 127 g in Form einer weißen mittelviskosen bis dickflüssigen Suspension mit einem pH-Wert von 11,13 entnommen. Die Probe wurde über Filterpapier auf der Nutsche problemlos filtriert und portionsweise mit 5 x 200 ml entionisiertem Wasser gewaschen. Der Filterkuchen wurde eine Stunde bei 120°C getrocknet. Röntgenometrisch wurde ein breiter Peak bei einem Schichtgitterabstand (D) von 3,8720 Å festgestellt, d.h. das Produkt war im wesentlichen noch amorph.

Nach 61 Stunden wurden 149 g einer zweiten Probe im Form einer weißen, mittelviskosen Suspension mit einem pH-Wert von 12,01 entnommen. Diese Probe wurde wie die erste Probe aufgearbeitet und zeigte ein Röntgenbeugungsspektrum mit einem sehr ausgeprägten Peak bei 3,8720 Å. Der Kristallinitätsgrad (Peakfläche/Untergrundfläche) betrug 96,8 %.

Nach 68 Stunden wurde die Synthese abgebrochen, und eine dritte Probe, die in der gleichen Weise wie die erste und die zweite Probe aufgearbeitet wurde, zeigte einen Kristallinitätsgrad von 97,9 %.

Der Rest des Ansatzes wurde auf größeren Nutschen filtriert, wobei 23,0 kg Filterkuchen und 12,47 kg Mutterlauge erhalten wurden. Die Mutterlauge hatte folgende Analyse: Si = 0,82 Gew.-%, Al = 5 ppm, Na = 1,22 Gew.-%, OH = 1,16 Gew.-%, Br = 3,71 Gew.-%, TPABr = 8,2 Gew.- %. Die Mutterlauge enthielt etwa 0,1 Gew.-% Impfkristalle mit einer mittleren Teilchengröße von etwa 30 - 40 nm und hatte einen pH-Wert von 12,25. Diese Mutterlauge wurde für die folgenden Ansätze verwendet.

Der Filterkuchen wurde in 120 Liter entionisiertem Wasser etwa 30 min. durch intensives Rühren homogenisiert, anschließend sedimentieren gelassen, und die überstehende Flüssigkeit wurde abdekantiert. Der Rückstand wurde 8 x mit je 100 Liter Wasser gewaschen, bis ein pH-Wert von 7,3 bis 7,4 erhalten wurde und das letzte Waschwasser eine elektrische Leitfähigkeit von 130 µS hatte. Der gesamte Waschvorgang dauerte etwa 5 Stunden, wobei 20,7 kg Filterkuchen erhalten wurden.

Der Filterkuchen wurde 48 Stunden bei 120°C getrocknet und auf einem Siebgranulator (Alexanderwerk) auf eine Teilchengröße von < 2,0 mm granuliert. Die Ausbeute an trockenen Granulaten betrug 6,3 kg.

Die Granulate wurden in einer Schichthöhe von 10 mm bei 540°C calciniert.

Die Ausbeute an calciniertem Produkt betrug 5,75 kg.

Charakterisierung:

GV 1000°C = 6,5 %; Si = 43,6 %; Al = 3,31 %; Na = 1,71 %; C = 182 ppm; Fe = 208 ppm; Ca = 257 ppm.

Das Si/Al-Verhältnis betrug 12,65, die BET-Oberfläche (nach DIN/66132) 352 m$^2$/g und die Kristallinität 96,96 %.

Es wurde auch der Anteil der bei üblichen Zeolithsynthesen auftretenden Fremdphasen durch Vergleich der Intensitäten der Röntgenbeugungslinien mit der höchsten Intensität bestimmt, und zwar für Mordenit ($D_{max}$ = 9,06 Å); Analcim ($D_{max}$ = 4,83 Å); und α-Cristobalit ($D_{max}$ = 3,345 Å). Das Verhältnis zwischen der Intensität der Röntgenbeugungslinie des erfindungsgemäßen Produktes mit der höchsten Intensität ($D_{max}$ = 3,8720 Å) und denen der drei Vergleichspeaks lag bei > 500 : 1. Das erfindungsgemäße Produkt ist also phasenrein.

Von den erhaltenen Produkt wurden Rasterelektronenmikroskop-(REM-) Aufnahmen angefertigt, wobei Kristallite mit einer mittleren Teilchengröße von etwa 30 nm gefunden wurden.

Die Kristallite waren im wesentlichen sphärisch, wobei das Verhältnis zwischem der langen und der kurzen Achse etwa 1,0 bis 1 : 1,5 betrug.

Vergleichsbeispiel 1

Die Arbeitsweise von Beispiel 1 wurde mit der Abweichung wiederholt, daß eine Mutterlauge mit 3,3 Gew.-% Impfkristallen mit einer Teilchengröße von 0,1 bis 1 µm verwendet wurde. Die Anzahl der Impfkristalle entsprach denen in der Mutterlauge nach Beispiel 1.

Der Versuch wurde nach 100 Stunden abgebrochen, da das dem Autoklaven entnommene Produkt keine Kristallinität zeigte (etwa vergleichbar der nach Beispiel 1 nach 20 Stunden entnommenen Probe).

Beispiel 2

Herstellung eines Zeolithen in der H-Form

Zur Herstellung eines Zeolithen in der H-Form wurden 3 300 g des nach Beispiel 1 erhaltenen calcinierten Produkts in einem 20-Liter-Gefäß mit Rührwerk mit 14,88 Liter entionisiertem Wasser vermischt. Die Suspension wurde unter Rühren auf 80°C erwärmt, worauf 1 624 g 37 %-ige Salzsäure zugesetzt wurden. Es wurde bei 80°C eine Stunde weitergerührt, worauf der Feststoff innerhalb von 30 min. sedimentieren gelassen wurde. Die überstehende Lösung wurde abgezogen, worauf nochmals 14,88 Liter entionisiertes Wasser und 1 624 g 37 %-ige Salzsäure zugegeben wurden. Die Suspension wurde unter Rühren auf 80°C erwärmt und eine Stunde bei 80°C gerührt. Anschließend wurde der Feststoff innerhalb von 2 min. sedimentieren gelassen. Die überstehende Lösung wurde abgezogen, und der Rückstand wurde mehrmals mit entionisiertem Wasser gewaschen, bis die Leitfähigkeit des Waschwassers unter 100 μS lag. Der Rückstand wurde anschliessend auf der Nutsche filtriert.

Der gewaschene Filterkuchen wurde 16 Stunden bei 120°C getrocknet, wobei 2,92 kg Trockenprodukt erhalten wurden. Das trockene Produkt wurde bei 540°C 10 Stunden calciniert. Die Ausbeute an calciniertem Produkt in der H-Form betrug 2,85 kg.

Das Produkt hatte folgende Eigenschaften:

Glühverlust = 6,7 Gew.-%; Si = 45,44 Gew.-%, Al = 2,71 Gew.-%, Na = 48 ppm; Fe = 80 ppm; C = 160 ppm.

Das Si/Al-Verhältnis betrug 16,1, verglichen mit 12,65 des Produkts nach Beispiel 1. Der Unterschied beruht darauf, daß bei der Säurebehandlung ein Teil des Aluminiums herausgelöst wurde.

Der Kristallinitätsgrad betrug 95,2 %. Die Lage des Hauptpeaks ($D_{max}$ = 3,856 Å) hatte sich praktisch nicht verändert. Auch die Kristallitgrößenverteilung und das Achsenverhältnis waren praktisch mit denen des Produkts von Beispiel 1 identisch.

Beispiel 3

Herstellung eines bindemittelhaltigen Granulats aus den Zeolithen in der H-Form von Beispiel 2

2200 g des Pulvers von Beispiel 2 wurden auf mit 3 500 g eines 25 %-igen Kieselsäuresols etwa 25 min. verrührt (Rührer mit 60 U/min.), bis eine plastische Masse erhalten wurde. Dieser Masse wurden zur Einstellung der Rheologie 44 g Hydroxymethyl-Hydroxypropylcellulose (Na-Salz) zugesetzt, worauf die Masse nochmals 10 min. bei 60 U/min. geknetet wurde. Die Masse wurde mit Hilfe eines Extruders (Händle PZVM8b) mit einem Mundstückdurchmesser von etwa 1,5 mm extrudiert. Die Extrudate wurden zu Stücken mit einer Länge von 3 mm geschnitten.

Die Extrudatstücke wurden zur Entfernung des Natriumgehalts (aus dem Cellulosederivat) einem Ionenaustausch mit Ammoniumnitrat unterzogen, wobei 2,44 kg der Extrudatstücke mit 4,89 Liter entionisiertem Wasser und 0,37 kg Amoniumnitrat bei 20 °C über einem Zeitraum von 1,5 Stunden vorsichtig verrührt wurden.

Nach dem Absetzen der Extrudatstücke wurde die überstehende Lösung abdekantiert. Die Extrudatstücke wurden mehrmals gewaschen. Nach der achten Wäsche lag die Leitfähigkeit des Waschwassers bei weniger als 70 μS. Die Extrudatstücke wurden 16 Stunden bei 120°C getrocknet, wobei die Ausbeute 2,40 kg betrug.

Das getrocknete Produkt wurde bei 600°C calciniert. Die Ausbeute an calciniertem Produkt betrug 2,35 kg.

Das calcinierte Produkt hatte folgende Eigenschaften:

Bindemittelgehalt 30 Gew.-% $SiO_2$, Na-Gehalt: 50 ppm; restliche Zusammensetzung wie nach Beispiel 2:

Kristallitätsgrad: 86,4 %
Seitendruckfestigkeit: 1,8 kp/3mm, mit einem Schleuniger-Modell 6 D bestimmt
Schüttgewicht: 550 g/Liter;
Porenvolumen (nach der Hg-Intrusionsmethode mit einem Porosimeter 4.000 bestimmt) = 0,36 cm$^3$/g

Porengrößenverteilung: > 1750 nm = 1,3 %, 1750 - 80 nm = 17,4 %; 80 bis 17 nm = 45,6 %; 14 bis 7,5 nm = 26,7 %.

Mittlerer Porenradius: 4 nm
spezifische Oberfläche (BET) 328 m$^2$/g.

Beispiel 4

Belegung des Granulats von Beispiel 3 mit Platin:

In 35 ml destilliertem Wasser wurden 0,287 g Hexachloroplatinsäure ($H_2PtCl_6$ = 10 Gew.-%Pt) unter Rühren aufgelöst. 100 g des Granulate von Beispiel 3 wurden in einem Probenglas mit dieser Lösung überschüttet und intensiv geschüttelt, so daß alle Granulate gleichmäßig benetzt wurden.

Die imprägnierten Granulate wurden 16 Stunden bei 120°C getrocknet und mit einer Aufheizrate von 1°C/min. an der Luft auf 450°C aufgeheizt, worauf diese Temperatur noch 5 Stunden aufrechterhalten wurde.

Vergleichsbeispiel 2

Nach der von S. Ernst und J. Weitkamp (Chem.Ing-Tech; 63; 748; 1991) beschriebenen Methode wurde eine Zeolithsynthese ohne Templat (TPABr) durchgeführt. Das Si/Al-Atomverhältnis in der Synthesemischung betrug 35 : 1. Als Rohstoffe kamen folgende Chemikalien zum Einsatz: Kolloidales Kieselsol (Syton X30; Monsanto); Destilliertes Wasser; Ätznatron technisch Prills (Hoechst); Natriumaluminat (Riedel de Haen).

Nach einer Reaktionszeit von 112 Stunden bei 160°C wurde ein mit ZSM-11 (D = 3,81 Å) und $\alpha$-Cristobalit ($D_{max}$ = 4,041 Å) verunreinigter MFI-Zeolith erhalten (Verhältnis der Peakintensitäten 100 : 59,76 bzw. 100 : 32,52. Im erfindungsgemäßen Beispiel lagen die Fremdphasenanteile unter der Nachweisgrenze von 100: 0,2.

Bei der REM-Untersuchung wurde eine Primärkristallitgröße von 0,5 bis 2 $\mu$m festgestellt. Die Primärkristallite hatten ein durchschnittliches Achsenverhältnis von 2,5 zu 1.

Beispiel 5

Herstellung von Zeolithen in der Na-Form:

1664 g demineralisiertes Wasser, 226,8 g Natriumhydroxid und 356 g Natriumaluminat wurden unter kräftigem Rühren auf 80°C erhitzt, bis eine klare Lösung erhalten wurde, die dann auf unter 60°C abgekühlt wurde.

In einem getrennten Gefäß wurden 22 kg einer Mutterlauge aus einem früheren Ansatz, die (analytisch) 3,43 Gew.-% $SiO_2$, 2,16 Gew.-% $Na_2O$ und 5,5 Gew.-% Tetrapropylammoniumbromid (TPABr) enthielt, vorgelegt. Die Mutterlauge enthielt ferner etwa 0,1 Gew.-% Impfkristalle mit einer mittleren Teilchengröße von 20 bis 50 nm. Die Impfkristalle hatten folgende (analytische) Zusammensetzung $SiO_2$; 91,77 Gew.-% $Al_2O_3$ 3,49 Gew.-%, Atomverhältnis Si/Al = 26,4.

Die Mutterlauge wurde mit 4280 g demineralisiertem Wasser verdünnt, worauf 1881 g TPABr und 6209 g Fällungskieselsäure (Silica FK320 Degussa) zugesetzt wurden. Das Gemisch wurde 30 bis 60 min. gerührt, worauf 2247 g der ersten Lösung zugesetzt wurden.

Das Gemisch wurde etwa 30 bis 60 min. homogenisiert. Der pH-Wert des Gels betrug 12,7.

Das Synthesegemisch wurde in einem 40 Liter-Autoklaven, der mit einem Rührwerk versehen war, eingefüllt und allmählich auf ca. 130°C erhitzt, wobei mit einer Rührdrehzahl von etwa 73 UpM gerührt wurde. Der Druck stieg während der Reaktion aufgrund der Zersetzung des TPABr allmählich von 2,6 bis auf 3,1 bar an.

Nach 63 Stunden wurde eine erste Probemenge von 143 g in Form einer weißen mittelviskosen Suspension mit einem pH-Wert von 12,05 entnommen. Die Probe wurde über Filterpapier auf der Nutsche filtriert und portionsweise mit 5 x 200 ml entionisiertem Wasser gewaschen. Der Filterkuchen wurde eine Stunde bei 120°C getrocknet. Die röntgenometrische Untersuchung der Probe zeigte einen ausgeprägten Peak bei 3,87 Å. Der Kristallinitätsgrad (Peakfläche/Untergrund) betrug 97,9 %.

Nach 80 Stunden wurde die Synthese abgebrochen, und eine zweite Probe, die in der gleichen Weise wie die erste Probe aufgearbeitet wurde, zeigte ebenfalls einen Kristallinitätsgrad von 97,9 %.

Dei Hauptmenge des Ansatzes wurde auf der Nutsche filtriert, wobei 16,7 kg Filterkuchen udn 19,5 kg Mutterlauge erhalten wurden. Die Mutterlauge wurde nicht näher untersucht.

Der Filterkuchen wurde in 120 Liter deionisiertem Wasser etwa 30 min. durch intensives Rühren homogenisiert, anschließend sedimentieren gelassen, und die überstehende Flüssigkeit wurde anschließend abdekantiert. Der Rückstand wurde 7 x mit je 100 Liter demineralisiertem Wasser gewaschen, bis ein pH-Wert von 7,7 bis 7,8 erhalten wurde und das letzte Waschwasser eine elektrische Leitfähigkeit von 170 $\mu$S hatte. Der gesamte Waschvorgang dauerte etwa 6 Stunden, wobei 16,35 kg Filterkuchen erhalten wurden.

Der Filterkuchen wurde 48 Stunden bei 120°C getrocknet und auf einem Siebgranulator (Alexanderwerk) auf eine Teilchengröße von < 2 mm granuliert. Die Ausbeute an trockenen Granulaten betrug 6,1 kg.

Die Granulate wurden in einer Schichthöhe von 10 mm bei 540°C calciniert.

Die Ausbeute an calciniertem Produkt betrug 5,4 kg.

Charakterisierung:

GV 1000°C = 5,9 Gew.-%; Si = 44,8 Gew.-%; Al = 1,86 Gew.-%; Na = 0,56 Gew.-%; C = 201 ppm
Das Si/Al-Verhältnis betrug 23,1, die BET-Oberfläche (nach DIN/66132) 379 $m^2$/g und die Kristallinität 97,8 %.

Die röntgenographische Untersuchung gab keine Hinweise auf Fremdphasen wie Mordenit, Analcim oder ZSM-11.

Von dem erhaltenen Produkt wurden rasterelektronenmikroskopische (REM-) Aufnahmen angefertigt, wobei Kristallite mit einer mittleren Teilchengröße von etwa 40 nm gefunden wurden. Die Kristallite waren sphärische ausgebildet, wobei das Verhältnis zwischen der langen und der kurzen Achse etwa 1.0 bis 1,5 betrug.

Beispiel 6

Herstellung von Zeolithen in der H-Form

Zur Herstellung eines Zeolithen in der H-Form wurden 2750 g des nach Beispiel 5 hergestellten Zeolithen in einem 20 Liter-Gefäß mit Rührwerk mit 12,4 kg deionisiertem Wasser vermischt. Die Suspension wurde unter Rühren auf 80°C erwärmt, worauf 1356 g Salpetersäure (62%-ig) zugegeben wurden. Nach einer Stunde Rühren bei 80°C wurde das Rührwerk abgestellt und der Feststoff sedimentieren gelassen. Die überstehende Lösung wurde abgezogen und der Rückstand mit mehreren Portionen deionisiertem Wasser gewaschen, bis die Leitfähigkeit des Waschwassers bei 100 μS lag. Der Rückstand wurde anschließend auf der Nutsche filtriert.

Der gewaschene Filterkuchen wurde 16 Stunden bei 120°C getrocket, wobei 2,625 kg Trockenprodukt erhalten wurden. Das getrocknete Produkt wurde bei 540°C 10 Stunden calciniert. Die Ausbeute an calciniertem Produkt in der H-Form betrug 2,580 kg.

Das Produkt wurde wie folgt charakterisiert:

Glühverlust = 3,9 Gew.-%; Si = 44,18 Gew.-%; Al = 1,62 Gew.-%; Na = 94 ppm.

Das Si/Al-Verhältnis betrug 26,2.

Die Kristallinität betrug 98,6 %. Die Lage des Hauptpeaks (Dmax = 3,856A) hatte sich praktisch nicht verändert. Auch die Kristallitgrößenverteilung und das Achsenverhältnis waren praktisch mit denen des Produkts von Beispiel 5 identisch.

Beispiel 7

Herstellung eines bindemittelhaltigen Granulats aus den Zeolithen in der H-Form von Beispiel 6:

2500 g des in Beispiel 6 hergestellten Zeolith-Pulvers wurden mit 734 g eines Böhmit-Pulvers (Aluminiumoxid) gemischt. Dieser Mischung wurden 203 g konzentrierte Essigsäure und 2400 g deionisiertes Wasser zugesetzt. Bei weiterem Mischen entstand so eine plastische Masse. Diese plastische Masse wurde noch weitere 30 min. gemischt, bevor sie mit Hilfe eines Händle-Extruders Typ PZVM8b mit einem Mundstück von ca. 1,5 mm extrudiert wurde. Die Extrudate wurden auf eine Länge von ca. 3 mm geschnitten.

Die erhaltenen Extrudate wurden bei 120°C 16 Stunden getrocknet und anschließend 5 Stunden bei 600°C calciniert.

Das erhaltene Produkt hatte folgende Eigenschaften:

Bindemittelgehalt 20 Gew.-% $Al_2O_3$; restliche Zusammensetzung wie nach Beispiel 6
Kristallinität:95,0
Seitendruckfestigkeit: 5,4 kp/3mm mit einem Schleuniger Modell 6 D bestimmt
Schüttgewicht: 591 g/Liter
Porenvolumen (nach Hg-Intrusionsmethode mit einem Porosimeter 4.000 bestimmt) = 0,36 $cm^3$/g
Porengrößenverteilung: > 1750 nm = 0.3 %; 1750 - 80 nm = 1,4 %; 80 - 17 nm = 14,8 %; 14 - 7,5 nm = 83,3 %
Mittlerer Porenradius: 6 nm
Spezifische Oberfläche (BET) = 330 $m^2$g

Anwendungsbeispiel 1

Anwendung des platinfreien Katalysators nach Beispiel 3 für die Isomerisierung von m-Xylol und die Hydrodealkylierung von

Ethylbenzol (EB) zu Benzol

In einen Edelstahlreaktor von 610 mm Länge und 19 mm Innendurchmesser und elektrischer Mantelheizung werden 15 ml des Katalysators von Beispiel 3 (verdünnt mit 15 ml inerten Glasperlen) eingefüllt.

Man aktiviert unter reinem Wasserstoff (>99,95 %) 12 Stunden bei 300°C. Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstroms wird gaschromatographisch bestimmt. Der Umsatz an Ethylbenzol (EB), der Xylolverlust und die Konzentration an p-Xylol gegenüber dem thermodynamischen Gleichgewichtswert (p-xylene approach to equililbrium, PXATE) werden berechnet.

| Zusammensetzung des Kohlenwasserstoffgemisches (KW) : | |
| --- | --- |
| Ethylbenzol (EB) | 15 Gew.-% |
| m-Xylol | 85 Gew.-% |

| Testbedingungen: | |
| --- | --- |
| Temperatur | 400°C |
| Druck | 28 barg |
| Raumgeschwindigkeit LHSV | 5,0 l/l/h |
| $H_2$/KW | 2,0 mol/mol |
| Laufzeit | 11 Stunden |

| Katalysatorleistung: | |
| --- | --- |
| Umsatz EB | 85,0 Gew.-% |
| Xylolverlust | 28,1 Gew.-% |
| PXATE | 97,7 % |

Berechnung:

$$\text{Umsatz EB \% = (EB ein - EB aus) x 100 /EB ein}$$

$$\text{Xylolverlust \% = (gesamt Xylole ein - gesamt Xylole aus) x}$$

$$\text{100 /gesamt Xylole ein}$$

$$\text{PXATE = (p-Xylol Isomer im Produkt normalisiert x 100 /p-Xylol}$$

$$\text{Isomer Gleichgewicht)}$$

Anwendungsbeispiel 2

Anwendung des platinhaltigen Katalysators von Beispiel 4 zur Xylolisomerisierung von m-Xylol und die Hydrodealkylierung von Ethylbenzol (EB) zu Benzol.

In einen Edelstahlreaktor von 610 mm Länge und 19 mm Innendurchmesser und elektrischer Mantelheizung werden 15 ml des Katalysators von Beispiel 4 (verdünnt mit 15 ml inerten Glasperlen) eingefüllt.
Man aktiviert unter reinem Wasserstoff (>99,95 %) 12 Stunden bei 300°C.
Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstroms wird gaschromatographisch bestimmt. Der Umsatz an Ethylbenzol (EB), der Xylolverlust und die Konzentration an p-Xylol gegenüber dem thermodynamischen Gleichgewichtswert (p-xylene approach to equilibrium, PXATE) werden berechnet.

| Zusammensetzung des Kohlenwasserstoffgemisches (KW): | |
| --- | --- |
| Ethylbenzol (EB) | 15 Gew.-% |
| m-Xylol | 85 Gew.-% |

| Testbedingungen: | |
| --- | --- |
| Temperatur | 400°C |
| Druck | 28 barg |
| Raumgeschwindigkeit LHSV | 5,0 l/l/h |
| $H_2$/KW | 2,0 mol/mol |
| Laufzeit | 15 Stunden |

| Katalysatorleistung: | |
| --- | --- |
| Umsatz EB | 96,0 Gew.-% |
| Xylolverlust | 14,5 Gew.-% |
| PXATE | 89,1 % |

Anwendungsbeispiel 3

Anwendung des platinhaltigen Katalysators nach Beispiel 4 für die Isomerisierung von m-Xylol und die Hydrodealkylierung von Ethylbenzol (EB) zu Benzol

In einen Edelstahlreaktor von 610 mm Länge und 19 mm Innendurchmesser und elektrischer Mantelheizung werden 15 ml des Katalysators von Beispiel 4 (verdünnt mit 15 ml inerten Glasperlen) eingefüllt.
Man aktiviert unter reinem Wasserstoff (>99,95 %) 12 Stunden bei 300°C.
Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstroms wird gaschromatographisch bestimmt. Der Umsatz an Ethylbenzol (EB), der Xylolverlust und die Konzentration an p-Xylol gegenüber dem thermodynamischen Gleichgewichtswert (p-xylene approach to equilibrium, PXATE) werden berechnet.

| Zusammensetzung des Kohlenwasserstoffgemisches (KW): | |
| --- | --- |
| Ethylbenzol (EB) | 20 Gew.-% |
| m-Xylol | 80 Gew.-% |

Die Testbedingungen und die Katalysatorleistung sind in Tabelle I zusammengefaßt.

Tabelle I

| Temperatur (°C) | Druck (barg) | LHSV (l/l/h) | $H_2$/KW (mol/mol) | Laufzeit (Std) | Umsatz EB (Gew%) | Xylolverlust (Gew%) | PXATE (%) |
| --- | --- | --- | --- | --- | --- | --- | --- |
| | | | | | | | |
| 360 | 8,0 | 3,0 | 3,0 | 360 | 86,1 | 9,0 | 101,6 |
| 390 | 8,0 | 5,0 | 3,0 | 49 | 95,3 | 8,8 | 101,6 |
| 360 | 8,0 | 10,0 | 3,0 | 293 | 57,9 | 4,4 | 101,7 |
| 380 | 8,0 | 10,0 | 3,0 | 188 | 74,5 | 6,4 | 101,8 |

Anwendungsbeispiel 4

Anwendung des platinfreien Katalysators nach Beispiel 3 für die Isomerisierung von Xylolen.

In einen Edelstahlreaktor von 290 mm Länge und 37 mm Innendurchmesser und elektrischer Mantelheizung werden 250 ml des Katalysators von Beispiel 3 eingefüllt.
Ein definiertes Kohlenwasserstoffgemisch der nachfolgend beschriebenen Zusammensetzung wird gemäß den nachfolgend beschriebenen Betriebsbedingungen (Testbedingungen) über das Katalysatorbett geleitet. Die Zusammensetzung des Produktstroms wird gaschromatographisch bestimmt.

| Testbedingungen: | |
|---|---|
| Temperatur | 260°C, 280°C, 300°C |
| Druck | 35 barg |
| Raumgeschwindigkeit WHSV | 10 g/g/h |

Die Zusammensetzung des eingetragenen Kohlenwasserstoffgemisches (KW) und die Produktverteilung sind in Tabelle II zusammengefaßt.

Tabelle II

| KW Bezeichnung | KW Eintrag (Gew%) | Produktverteilung (Gew%) | | |
|---|---|---|---|---|
| | | 260 °C | 280 °C | 300 °C |
| | | | | |
| Nicht-Aromaten | 0,3 | 0,3 | 0,3 | 0,4 |
| Benzol | 0,1 | 0,1 | 0,1 | 0,1 |
| Toluol | 0,4 | 0,6 | 1,1 | 1,6 |
| EB | 0,3 | 0,3 | 0,3 | 0,3 |
| p-Xylol | 11,4 | 21,5 | 22,7 | 22,6 |
| m-Xylol | 54,3 | 50,5 | 50,7 | 50,2 |
| o-Xylol | 31,3 | 24,6 | 22,4 | 21,8 |
| $C_9^+$ Aromaten | 1,9 | 2,1 | 2,4 | 3,0 |
| EB : Ethylbenzol | | | | |

Anwendungsbeispiel 5

Anwendung des Katalysators nach Beispiel 7 für die Disproportionierung von Toluol.

In einen Edelstahlreaktor von 610 mm Länge und 19 mm Innendurchmesser und elektrischer Mantelheizung werden 15 ml des Katalysators von Beispiel 7 (verdünnt mit 15 ml inerten Glasperlen) eingefüllt.
Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstroms wird gaschromatographisch bestimmt.

| Testbedingungen: | |
|---|---|
| Temperatur | 400°C, 440°C, 480°C |
| Druck | 15 barg |
| Raumgeschwindigkeit LHSV | 0,5 l/l/h |
| $H_2$/KW | 3,0 mol/mol |

Die Zusammensetzung des eingetragenen Kohlenwasserstoffgemisches (KW) und die Produktverteilung sind in Tabelle III zusammengefaßt.

Tabelle III

| KW Bezeichnung | KW Eintrag (Gew%) | Produktverteilung (Gew%) | | |
|---|---|---|---|---|
| | | 400 °C | 440 °C | 480 °C |
| | | | | |
| Nicht-Aromaten | 0,0 | 0,1 | 0,1 | 0,0 |
| Benzol | 0,0 | 8,2 | 15,5 | 31,8 |

EP 1 230 157 B1

Tabelle III   (fortgesetzt)

| KW Bezeichnung | KW Eintrag (Gew%) | Produktverteilung (Gew%) | | |
|---|---|---|---|---|
| | | 400 °C | 440 °C | 480 °C |
| Toluol | 99,5 | 80,1 | 60,3 | 41,2 |
| EB | 0,1 | 0,1 | 0,5 | 0,0 |
| p-Xylol | 0,1 | 2,6 | 5,0 | 5,4 |
| m-Xylol | 0,2 | 5,6 | 11,0 | 12,0 |
| o-Xylol | 0,1 | 2,4 | 5,0 | 5,3 |
| $\Sigma\, C_8$ | 0,5 | 10,7 | 21,5 | 22,7 |
| $C_9$ Aromaten | 0,0 | 0,9 | 2,5 | 3,9 |
| $C_{10}^+$ Aromaten | 0,0 | 0,0 | 0,1 | 0,4 |
| EB : Ethylbenzol | | | | |

Anwendungsbeispiel 6

Anwendung des Katalysators nach Beispiel 7 für die Transalkylierung substituierter Aromaten

In einen Edelstahlreaktor von 610 mm Länge und 19 mm Innendurchmesser und elektrischer Mantelheizung werden 15 ml des Katalysators von Beispiel 7 (verdünnt mit 15 ml inerten Glasperlen) eingefüllt.

Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstroms wird gaschromatographisch bestimmt.

| Testbedingungen: | |
|---|---|
| Temperatur | 440°C, 480°C |
| Druck | 15 barg |
| Raumgeschwindigkeit LHSV | 0,5 l/l/h |
| $H_2$/KW | 3,0 mol/mol |

Die Zusammensetzung des eingetragenen Kohlenwasserstoffgemisches (KW) und die Produktverteilung sind in Tabelle IV zusammengefaßt.

Tabelle IV

| KW Bezeichnung | KW Eintrag (Gew%) | Produktverteilung (Gew%) | | |
|---|---|---|---|---|
| | | | 440 °C | 480 °C |
| | | | | |
| Nicht-Aromaten | 0,3 | | 0,8 | 0.1 |
| Benzol | 0,0 | | 14,2 | 15,9 |
| Toluol | 0,0 | | 38,3 | 41,2 |
| EB | 0,0 | | 1,5 | 1,5 |
| p-Xylol | 0,0 | | 6,6 | 6,6 |
| m-Xylol | 0,1 | | 14,5 | 14,6 |
| o-Xylol | 0,4 | | 6,5 | 6,7 |
| $\Sigma\, C_8$ | 0,5 | | 29,1 | 29,4 |
| $C_9$ Aromaten | 93,8 | | 15,2 | 11,2 |
| $C_{10}^+$ Aromaten | 5,4 | | 2,4 | 2,2 |

12

Anwendungsbeispiel 7

Anwendung des Katalysators nach Beispiel 7 für die Umwandlung von Methanol in höhere Kohlenwasserstoffe

In einem 2-Reaktor-System, bestehend aus dem Dimethylether-(DME)-Vorreaktor R1 und dem DME-Umwandlungsreaktor R2 wird ein Gemisch aus Methanol und Wasser in eine Mischung aus (Wasser und) Kohlenwasserstoffen, bestehend aus Olefinen, Paraffinen und Aromaten, umgewandelt. Die Umwandlung von Methanol zu DME und Wasser erfolgt im DME-Vorreaktor R1 mittels eines sauren Katalysators, in der Regel $\gamma$-Al$_2$O$_3$. Die Umwandlung von DMEzu höheren Kohlenwasserstoffen erfolgt im DME-Umwandlungsreaktor R2 über dem in Beispiel 7 beschriebenen Katalysator.

In den Edelstahlreaktor R1 von 627 mm Länge und 33,5 mm Innendurchmesser und elektrischer Mantelheizung werden 100 ml handelsüblicher $\gamma$-Al$_2$O$_3$ Tabletten (Süd-Chemie AG, 4,5 x 4,5 mm) eingefüllt. In den Edelstahlreaktor R2 von 618 mm Länge und 33,5 mm Innendurchmesser und elektrischer Mantelheizung werden 50 ml des Katalysators von Beispiel 7 eingefüllt.

Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Stickstoff (>99,996 %) über das Katalysatorbett geleitet. Die Zusammensetzung des Produktstroms wird gaschromatographisch bestimmt. Die Produktverteilung ist in Tabelle V zusammengefaßt.

| Zusammensetzung des Kohlenwasserstoff/Wasser-Gemisches (KW): | |
|---|---|
| Methanol | 80 Gew.-% |
| Wasser | 20 Gew.-% |

| Testbedingungen: | |
|---|---|
| Temperatur R1 | 320°C |
| Temperatur R2 | 300°C, 360°C |
| Druck | 20 barg |
| Raumgeschwindigkeit WHSV | 2,0 g/g/h |
| N$_2$/KW | 9,0 mol/mol |
| Umsatz Methanol | >98 % |

Tabelle V

| KW Bezeichnung | | Produktverteilung (Gew%) | |
|---|---|---|---|
| | | 300°C | 360°C |
| | | | |
| C$_1$-C$_4$ | | 36 | 50 |
| C$_5$-C$_8$ (PON) | | 33 | 22 |
| Aromaten und C$_9^+$ | | 31 | 28 |
| C$_5^+$ | | 64 | 50 |
| PON : Paraffine, Olefine, Naphihene | | | |

Anwendungsbeispiel 8

Anwendung des Katalysators nach Beispiel 7 für die Oligomerisierung von Olefinen

In einen Edelstahlreaktor von 500 mm Länge und 23 mm Innendurchmesser und elektrischer Mantelheizung werden 19 ml des Katalysators von Beispiel 7 (verdünnt mit 19 ml inerten Glasperlen) eingefüllt.

Ein definiertes Kohlenwasserstoffgemisch der nachfolgend beschriebenen Zusammensetzung wird gemäß den nachfolgend beschriebenen Betriebsbedingungen (Testbedingungen) zusammen mit reinem Wasserstoff (>99,95 %) über das Katalysatorbett geleitet. Die Zusammensetzung des Produktstrofms wird gaschromatographisch bestimmt.

| Testbedingungen: | |
|---|---|
| Temperatur | 230°C |
| Druck | 75 barg |
| Raumgeschwindigkeit WHSV | 1,0 g/g/h |
| Laufzeit : Umsatz $\Sigma_{Butene}$ | 71 Stunden und 168 Stunden >99 mol% |

Die Zusammensetzung des eingetragenen Kohlenwasserstoffgemisches (KW) und die Produktverteilung der Flüssigphase sind in Tabelle VI zusammengefaßt.

Tabelle VI

| KW Bezeichnung | KW Eintrag | Produktverteilung Flüssigphase (Gew%) | | |
|---|---|---|---|---|
| | | | 71 Std | 168 Std |
| | | | | |
| $C_1$-$C_3$ | 0,4 | <$C_8$ | 6 | 6 |
| i-Butan | 32,0 | $C_8$ | 12 | 23 |
| n-Butan | 9,0 | >$C_8$-$C_{12}$ | 37 | 40 |
| l-Buten | 14,0 | >$C_{12}$-$C_{16}$ | 26 | 22 |
| cis/trans-Buten | 29,0 | >$C_{16}$-$C_{20}$ | 12 | 7 |
| i-Buten | 15,0 | >$C_{20}$ | 7 | 2 |
| andere | 0,6 | | | |
| | | | | |
| | | | | |
| | | | | |

## Patentansprüche

1.  Verfahren zur Herstellung on synthetischen Zeolithen mit MFI-Struktur, einem Si/Al-Atomverhältnis von etwa 8 bis 45 und sehr kleinen Primärkristalliten, wobei man eine Si-Quelle, eine Al-Quelle und ein organisches Templat unter hydrothermalen Bedingungen miteinander umsetzt, wobei das Si/Al-Atomverhältnis etwa 9 bis 50 und das Verhältnis zwischen längster und kürzester Achse der primärkristallite etwa 1,0 bis 1,5 : 1 beträgt, **dadurch gekennzeichnet, dass** man die Umsetzung in Gegenwart von Impfkristallen mit einer mittleren Teilchengröße von etwa 0,01 bis 0,1μm; vorzugsweise von 0,02 bis 0,05μm, aus einem früheren Ansatz ohne Abtrennung von der Mutterlauge und ohne Anwendung von Keimbildungsgelen durchführt, wobei die Primärkristallite hochkristallin sind, eine hohe spezifische Oberfläche und eine Größe von etwa 0,01 bis 0,05μm haben.

2.  Verfahren nach Anspruch 1, **dadurch gekennzeichnet, daß** man als Si-Quelle Fällungskieselsäure, als Al-Quelle Natriumaluminat und als organisches Templat Tetrapropylammoniumbromid verwendet.

3.  Verfahren nach einem der Ansprüche 1 bis 2, **dadurch gekennzeichnet, daß** man die hydrothermale Umsetzung bei einer Temperatur von etwa 100 bis 180° C, bei einem pH-Wert von etwa 11 bis 13 und bei einem Gewichtsverhältnis zwischen Siund Al-Quelle von etwa 2,95 bis 26,5 durchführt.

4.  Verfahren nach einem der Ansprüche 1 bis 3, **dadurch gekennzeichnet, daß** die Konzentration der Impfkristalle im Reaktionsansatz etwa 0,1 bis 0,2 Gew.-% beträgt.

5.  Verfahren nach einem der Ansprüche 1 bis 4, **dadurch gekennzeichnet, daß** man das Reaktionsprodukt trocknet und calciniert.

6. Synthetische Zeolithen mit MFI-Struktur, erhältlich nach dem Verfahren nach einem der Ansprüche 1 bis 5, **gekennzeichnet durch** die Kombination folgender Merkmale:

   (a) Si/Al- Atomverhältnis: etwa 8 bis 45 : 1
   (b) Größe der Primärkristallite: etwa 0,01 bis 0,05 $\mu$m;
   (c) Verhältnis zwischen längster und kürzester Achse der Primärkristallite: etwa 1,0 bis 1,5 : 1;
   (d) Verhältnis zwischen der Intensität der Röntgenbeugungslinie mit der höchsten Intensität ($D_{max}$ = 3,865 $\pm$ 0,015 Å) und der Intensität der Röntgenbeugungslinien von ZSM 11-Zeolith ($D_{max}$ = 11,15 Å); Mordenit ($D_{max}$ = 9,06 Å,) $\alpha$-Cristobalit ($D_{max}$ = 3,345 Å) und Analcim ($D_{max}$ = 4,83 Å) > 500 : 1.

7. Verfahren nach einem der Ansprüche 1 bis 5, **dadurch gekennzeichnet, daß** man das Reaktionsprodukt durch Ionenaustausch in die H-Form überführt, unter Zusatz von Bindemitteln Formkörper erzeugt und vor oder nach der Erzeugung der Formkörper gegebenenfalls katalytisch wirksamen Unedelmetall- und/oder Edelmetallkomponenten zusetzt.

8. Verwendung der synthetischen Zeolithe nach Anspruch 6 oder hergestellt nach einem der Ansprüche 1 bis 5 und 7 als Katalysatoren.

9. Verwendung nach Anspruch 8 für säurekatalysierte Reaktionen, Oxidation, Reduktion und Adsorption.

10. Verwendung nach Anspruch 8 als Katalysator für die Xylolisomerisierung.


**Claims**

1. A method for the preparation of synthetic zeolites with an MFI structure, with an Si/Al atomic ratio of approximately 8 to 45 and very small primary crystallites, wherein an Si source, an Al source and an organic template are reacted together under hydrothermal conditions, wherein the Si/Al atomic ratio is approximately 9 to 50 and the ratio between the longest and shortest axis of the primary crystallites is approximately 1.0 to 1.5:1, **characterised in that** the reaction is carried out in the presence of seed crystals with a mean particle size of approximately 0.01 to 0.1 $\mu$m, preferably 0.02 to 0.05 $\mu$m, from a previous batch without separation of the mother liquor and without using nucleating gels, wherein the primary crystallites are highly crystalline, have a high specific surface and are of a size of approximately 0.01 to 0.05 $\mu$m.

2. A method according to Claim 1, **characterised in that** precipitation silicic acid is used as the Si source, a sodium aluminate is used as the Al source and tetrapropylammonium bromide is used as the organic template.

3. A method according to either one of Claims 1 and 2, **characterised in that** the hydrothermal reaction is carried out at a temperature of approximately 100 to 180°C, at a pH value of approximately 11 to 13 and at a weight ratio between the Si source and the Al source of approximately 2.95 to 26.5.

4. A method according to any one of Claims 1 to 3, **characterised in that** the concentration of the seed crystals in the reaction batch is approximately 0.1 to 0.2 % by weight.

5. A method according to any one of Claims 1 to 4, **characterised in that** the reaction product is dried and calcined.

6. Synthetic zeolites with an MFI structure, which can be obtained in accordance with the method according to any one of Claims 1 to 5, **characterised by** a combination of the following features:

   (a) Si/Al atomic ratio approximately 8 to 45:1
   (b) size of the primary crystallites: about 0.01 to 0.05 $\mu$m;
   (c) ratio between the longest and shortest axis of the primary crystallites: about 1.0 to 1.5:1;
   (d) ratio between the intensity of the X-ray diffraction line with the highest intensity ($D_{max}$ = 3.865 $\pm$ 0.015 Å) and the intensity of the X-ray diffraction lines of ZSM 11 zeolite ($D_{max}$ = 11.15 Å); mordenite ($D_{max}$ = 9.06 Å) $\alpha$-crystobalite ($D_{max}$ = 3.345 Å) and analcim ($D_{max}$ = 4.83 Å) > 500:1.

7. A method according to any one of Claims 1 to 5, **characterised in that** the reaction product is converted by ion exchange into the H form, mouldings are produced with the addition of binders and before or after the production of the mouldings optionally catalytically active base metal and/or noble metal components are added.

**8.** Use of the synthetic zeolites according to Claim 6 or prepared according to any one of Claims 1 to 5 and 7 as catalysts.

**9.** Use according to Claim 8 for acid-catalysed reactions, oxidation, reduction and adsorption.

**10.** Use according to Claim 8 as a catalyst for xylene isomerisation.

## Revendications

**1.** Procédé pour la préparation de zéolithes synthétiques à structure MFI, un rapport atomique Si/Al d'environ 8 à 45 et des cristaux primaires très petits, dans lequel on transforme l'un avec l'autre une source de Si, une source de Al et une matrice organique sous des conditions hydrothermiques, le rapport atomique Si/Al étant d'environ 9 à 50 et le rapport entre l'axe le plus long et le plus court des cristaux primaires étant d'environ 1,0 à 1,5:1, **caractérisé en ce qu'**on réalise la transformation en présence de cristaux d'ensemencement présentant une taille moyenne des particules d'environ 0,01 à 0,1 μm, de préférence de 0,02 à 0,05 μm, d'une charge précédente sans séparation de la lessive-mère et sans utilisation de gels de formation de germes, les cristaux primaires étant hautement cristallins, présentant une surface spécifique élevée et une grosseur d'environ 0,01 à 0,05 μm.

**2.** Procédé selon la revendication 1, **caractérisé en ce qu'**on utilise comme source de Si des silices précipitées, comme source d'Al de l'aluminate de sodium et comme matrice organique du bromure de tétrapropylammonium.

**3.** Procédé selon l'une quelconque des revendications 1 et 2, **caractérisé en ce qu'**on effectue la transformation hydrothermique à une température d'environ 100 à 180°C, à un pH d'environ 11 à 13 et à un rapport pondéral entre la source de Si et la source d'Al d'environ 2,95 à 26,5.

**4.** Procédé selon l'une quelconque des revendications 1 à 3, **caractérisé en ce que** la concentration en cristaux d'ensemencement dans la charge réactionnelle est d'environ 0,1 à 0,2% en poids.

**5.** Procédé selon l'une quelconque des revendications 1 à 4, **caractérisé en ce qu'**on sèche le produit de réaction et qu'on le calcine.

**6.** Zéolithes synthétiques à structure MFI, pouvant être obtenues selon le procédé selon l'une quelconque des revendications 1 à 5, **caractérisées par** la combinaison des caractéristiques suivantes :

(a) rapport atomique Si/Al : environ 8 à 45/1
(b) grosseur des cristaux primaires : environ 0,01 à 0,05 μm
(c) rapport entre l'axe le plus long et l'axe le plus court des cristaux primaires : environ 1,0 à 1,5:1
(d) rapport entre l'intensité de la ligne de diffraction de rayons X présentant l'intensité la plus élevée ($D_{max}$ = 3,865 ± 0,015 Å) et l'intensité de la ligne de diffraction de rayons X de la zéolithe ZSM 11 ($D_{max}$ = 11,15 Å) ; la mordénite ($D_{max}$ = 9,06 Å), la cristobalite ($D_{max}$ = 3,345 Å) et l'analcime ($D_{max}$ = 4,83 Å) > 500:1.

**7.** Procédé selon l'une quelconque des revendications 1 à 5, **caractérisé en ce qu'**on transforme le produit de réaction par échange ionique dans la forme H, qu'on obtient des corps façonnés en ajoutant des liants et qu'on ajoute, avant ou après l'obtention des corps façonnés, des composants métalliques non précieux et/ou des composants métalliques précieux le cas échéant catalytiquement actifs.

**8.** Utilisation des zéolithes synthétiques selon la revendication 6 ou préparées selon l'une quelconque des revendications 1 à 5 et 7 comme catalyseurs.

**9.** Utilisation selon la revendication 8 pour des réactions, oxydation, réduction et adsorption catalysées par des acides.

**10.** Utilisation selon la revendication 8 comme catalyseur pour l'isomérisation du xylène.